# EUROPEAN PATENT APPLICATION

(11) **EP 4 778 914 A1**
(43) Date of publication of application: **22.07.2026**
(21) Application number: 24864394.2
(22) Date of filing: 19.08.2024
(51) Int. Cl.: C07C 213/08, C07C 217/84, C07C 269/04, C07C 237/20

(54) **METHOD FOR SYNTHESIZING ELACESTRANT AND INTERMEDIATE THEREOF**

(30) Priority: 13.09.2023 CN 202311178672
(71) Applicant: Aurisco Pharmaceutical co., Ltd., Zhejiang 317200 (CN); Aurisco Pharamceutical (Tianjin) Inc, Binhai New Area, Tianjin 300457 (CN)
(72) Inventor: GUO, Wancheng, Tianjin 300457 (CN); FANG, Jie, Tianjin 300457 (CN); CHENG, Kun, Tianjin 300457 (CN); CHU, Dingjun, Taizhou, Zhejiang 317200 (CN); XIE, Xiaoqiang, Taizhou, Zhejiang 317200 (CN)
(74) Representative: Mewburn Ellis LLP
(86) International application number: PCT/CN2024/113070
(87) International publication number: WO 2025/055671

(57) **Abstract**

The present invention provides a method for synthesizing Elacestrant, including reacting an amino compound, i.e., a compound of Formula V, with a chiral intermediate, i.e., a compound of Formula VI, reducing a generated product by a reducing agent that can provide an ethyl group, so as to generate a compound of Formula VII, and then removing a protecting group to afford Elacestrant, where a reaction equation is as follows. According to the method for synthesizing Elacestrant of the present invention, the risks of low yield and substrate racemization caused by amide reduction are avoided, the yield is high, the material cost is low, the operation is safe, and the present invention is suitable for industrial production.

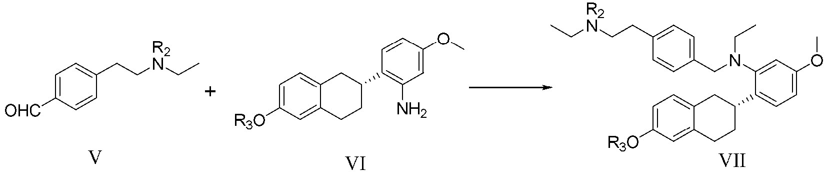

## Description

The present application claims the priority of Chinese Patent Application No. 202311178672.7, filed on September 13, 2023, the entire content of which is incorporated herein by reference.

### TECHNICAL FIELD

The present invention belongs to the field of active pharmaceutical ingredient synthesis, and more specifically, relates to a method for synthesizing Elacestrant and an intermediate thereof.

### BACKGROUND

Elacestrant is an ER degrader (SERD) developed by Menarini, and is used for the treatment of postmenopausal women or adult men with ER+, HER2-, ESR1-mutated advanced or metastatic breast cancer whose disease has progressed following at least first-line endocrine therapy. It was approved for marketing by the FDA on January 27, 2023, under the brand name ORSERDU (oral tablets, 86 mg and 345 mg). Its structure is expressed by the following formula:

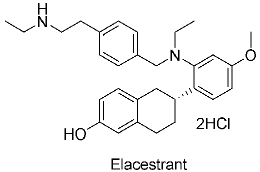

Regarding the synthetic methods for Elacestrant, only two routes have been reported by the originator company thus far. The first route is the synthetic route reported in the prior art WO2004058682.

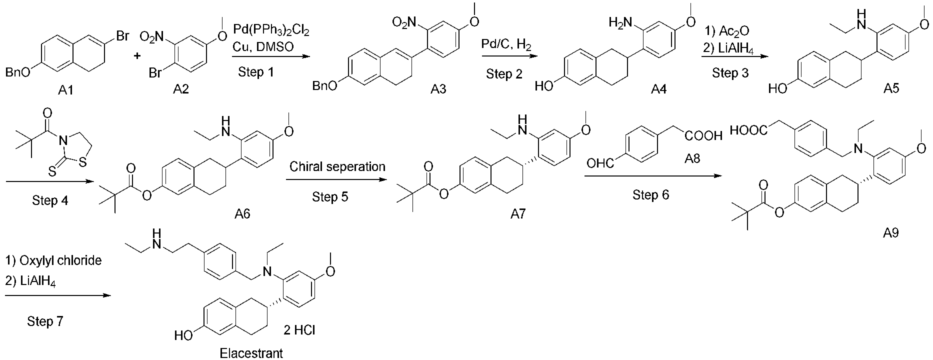

In this route, compounds A1 and A2 undergo a coupling reaction to afford a compound A3, which is then subjected to Pd/C catalytic reduction to afford a compound A4. Compound A4 is a racemate and requires chiral separation in the fifth step, with a maximum yield of only 50%. Both the third and seventh steps of this route involve the reduction of amide groups using lithium aluminum hydride to afford the corresponding amines, which not only results in low yields but also employs the hazardous reagent lithium aluminum hydride. Furthermore, the raw material A8 used is non-conventional. It is not available as an industrial product on the market. Moreover, there is no suitable synthetic method for its production at present.

In 2020, a second route was reported in the prior art WO2020167855.

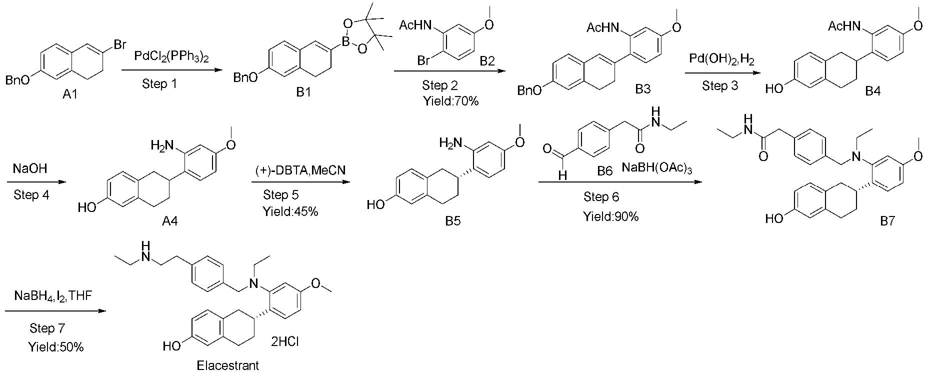

Compared to the first route, this route shows a significant improvement in the final product yield. However, the overall yield remains relatively low (total yield 12.75%). This is attributed to the fact that the route still requires chiral separation to afford a single-configuration intermediate, with the yield of the fifth-step resolution reaction being only 45%. Additionally, the final step involving amide reduction yields only 50% after purification. Due to the chiral nature of the substrate and the presence of sensitive functional groups, the choice of reducing agents is limited, posing a risk of racemization in the product. Moreover, the raw material B6 is non-conventional and is not available as an industrial product on the market.

In summary, the synthetic methods for Elacestrant in the prior art fail to achieve asymmetric reduction, resulting in low yields. Furthermore, these methods employ hazardous reagents and specialized equipment, rendering them unsuitable for industrialization and leading to high product costs. Therefore, there is an urgent need in the art to develop a low-cost, high-yield, operationally simple, and industrially suitable method for synthesizing Elacestrant.

### SUMMARY

Aiming at the defects in the methods for synthesizing Elacestrant in the prior art, the present invention provides a novel method for synthesizing Elacestrant, including the following steps:
(1) reacting a compound of Formula V with a compound of Formula VI, and reducing a generated product by a reducing agent to generate a compound of Formula VII, where the reducing agent further provides an ethyl group, and a reaction equation is as follows: 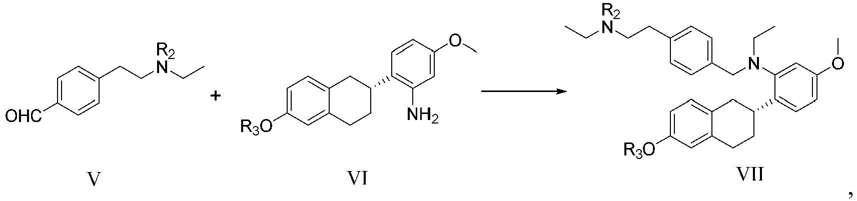 where in the reaction equation, R₂ is a nitrogen protecting group, and R₃ is H or a hydroxy protecting group; and
(2) removing a nitrogen protecting group from the compound of Formula VII to afford Elacestrant, where the protecting group is a nitrogen protecting group and a hydroxy protecting group, or is a nitrogen protecting group.

In another preferred example, in step (1), the compound of Formula V reacts with the compound of Formula VI at 50°C to 80°C to generate a Schiff base, then the Schiff base is reduced by the reducing agent at 40°C to 70°C, and an ethyl group is introduced to afford the compound of Formula VII.

In another preferred example, the reaction for generating the Schiff base is carried out in a solvent selected from toluene, tetrahydrofuran, methyltetrahydrofuran, or a combination thereof. In another preferred example, the reaction for reducing the Schiff base and introducing the ethyl group is carried out in a solvent selected from toluene, tetrahydrofuran, methyltetrahydrofuran, or a combination thereof.

In another preferred example, a molar ratio of the compound of Formula VI to the compound of Formula V ranges from 1:1 to 1.2.

In another preferred example, in step (1), the reducing agent used in the reaction is selected from sodium triacetoxyborohydride.

In another preferred example, the nitrogen protecting group is selected from Boc or Fmoc.

In another preferred example, the hydroxy protecting group is selected from Bn, Cbz, or Ac.

In another preferred example, in step (2), when R₃ is H, the nitrogen protecting group is removed from the compound of Formula VII to afford Elacestrant. In another preferred example, in step (2), when R₃ is a hydroxy protecting group, the nitrogen protecting group and the hydroxy protecting group are sequentially removed from the compound of Formula VII, or the hydroxy protecting group and the nitrogen protecting group are sequentially removed from the compound of Formula to afford Elacestrant.

In another preferred example, in the method for synthesizing Elacestrant, a method for synthesizing the compound of Formula V includes the following step: converting R₁ in the compound of Formula IV into an aldehyde group to afford the compound of Formula V, where a reaction equation is as follows: 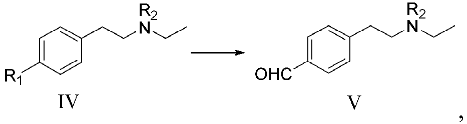 where in the reaction equation, R₁ is selected from bromine or iodine, and R₂ is a nitrogen protecting group.

In another preferred example, converting R₁ in the compound of Formula IV into an aldehyde group is carried out in the presence of a strong base reagent, and the strong base reagent is selected from n-butyllithium, tert-butyllithium, or lithium metal, preferably n-butyllithium,

In another preferred example, converting R₁ in the compound of Formula IV into an aldehyde group is carried out in the presence of a metal catalyst, the metal catalyst is a palladium-containing catalyst, and the palladium-containing catalyst selected from palladium acetate, tetrakis(triphenylphosphine)palladium, Pd(dppf)Cl₂, or Pd(dba)₂.

According to another aspect, the present invention provides a method for synthesizing a compound of Formula V, including the following steps:
(1) subjecting a compound of Formula I to a condensation reaction with ethylamine to afford a compound of Formula II;
(2) reducing the compound of Formula II to afford a compound of Formula III;
(3) protecting an amino group in the compound of Formula III to afford a compound of Formula IV; and
(4) converting R₁ in the compound of Formula IV into an aldehyde group to afford a compound of Formula V,

where a reaction equation is as follows: 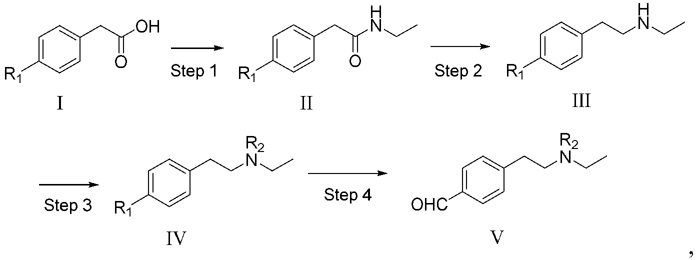
where in the reaction equation, R₁ is selected from bromine or iodine, and R₂ is a nitrogen protecting group.

According to yet another aspect, the present invention provides a method for synthesizing Elacestrant, including the following steps:
(1) reacting a compound of Formula V with a compound of Formula VI, and reducing a generated product by a reducing agent to generate a compound of Formula VII, where the reducing agent further provides an ethyl group, and a reaction equation is as follows: 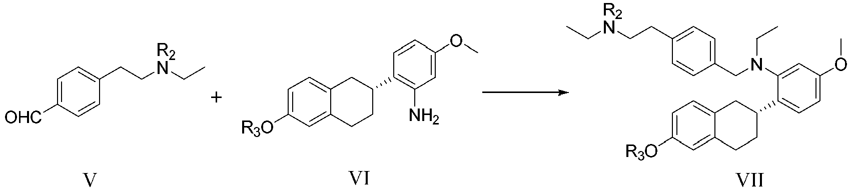 where in the reaction equation, R₂ is a nitrogen protecting group, and R₃ is H or a hydroxy protecting group; and
(2) removing a protecting group from the compound of Formula VII to afford Elacestrant, where the protecting group is a nitrogen protecting group and a hydroxy protecting group, or is a nitrogen protecting group,
where the compound of Formula V is obtained by adopting the method for synthesizing the compound of Formula V.

### BRIEF DESCRIPTION OF THE DRAWINGS

FIG. 1 shows an ¹H-NMR spectrum of a compound of Formula II-1 obtained in Example 1.
FIG. 2 shows an ¹H-NMR spectrum of a compound of Formula III-1 obtained in Example 1.
FIG. 3 shows an ¹H-NMR spectrum of a compound of Formula IV-1 obtained in Example 1.
FIG. 4 shows an ¹H-NMR spectrum of a compound of Formula V-1 obtained in Example 1.
FIG. 5 shows an ¹H-NMR spectrum of a compound of Formula VII-1 obtained in Example 2.
FIG. 6 shows an HPLC spectrum of the compound of Formula VII-1 obtained in Example 2.
FIG. 7 shows an ¹H-NMR spectrum of a compound Elacestrant obtained in Example 2.
FIG. 8 shows an HPLC spectrum of the compound Elacestrant obtained in Example 2.
FIG. 9 shows a mass spectrum of the compound Elacestrant obtained in Example 2.

### DETAILED DESCRIPTION

In response to the defects in the methods for synthesizing Elacestrant in the prior art, the inventors of the present application, through in-depth research, have found that in the synthesis of Elacestrant, the use of an amide-free intermediate, i.e., a compound of Formula V, together with a chiral intermediate containing a tetrahydronaphthalene ring, i.e., a compound of Formula VI, via reductive amination to afford the Elacestrant structure, avoids the risks of low yield and substrate racemization caused by amide reduction. This synthetic method is characterized by high yield across the entire route and low cost, significantly reducing the API cost.

### Method for Synthesizing Compound of Formula V

In the present invention, a method for synthesizing a compound of Formula V includes the following steps:
(1) subjecting a compound of Formula I to a condensation reaction with ethylamine to afford a compound of Formula II;
(2) reducing the compound of Formula II to afford a compound of Formula III;
(3) protecting an amino group in the compound of Formula III to afford a compound of Formula IV; and
(4) converting R₁ in the compound of Formula IV into an aldehyde group to afford the compound of Formula V,

where a reaction equation is as follows: 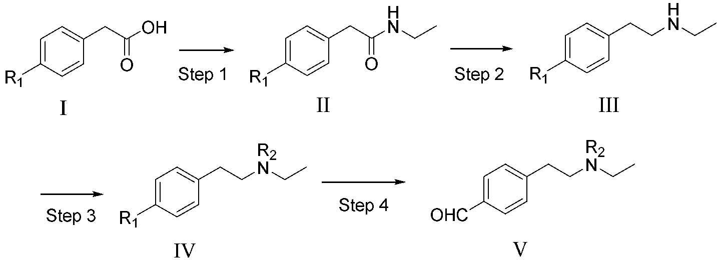
where in the reaction equation, R₁ is bromine or iodine, and R₂ is a nitrogen protecting group.

In step (1), the condensing agent used in the condensation reaction is a condensing agent commonly used for such reactions, and may be selected from EDCI, DCC, HOBt, CDI, HBTu, or a combination thereof. The reaction solvent is a solvent commonly used for such reactions, and may be selected from dichloromethane, N,N-dimethylformamide, dimethyl sulfoxide, toluene, or tetrahydrofuran, preferably selected from a mixed solvent of dichloromethane and N,N-dimethylformamide. A molar ratio of the reaction raw material ethylamine to the compound of Formula I preferably ranges from 0.8-4:1, and more preferably 1.2:1. During the material addition process, the temperature of the reaction system is controlled at 0°C to 15°C. Upon completion of material addition, the reaction system is allowed to react at room temperature until the compound of Formula I is completely converted.

In step (2), the reducing agent used for the amide reduction is a reducing agent commonly used for such reactions, and may be selected from borane, a borane complex, lithium aluminum hydride, sodium borohydride, sodium borohydride/Lewis acid, or a combination thereof, more preferably borane-dimethyl sulfide complex and/or lithium aluminum hydride. The reaction solvent is a solvent commonly used for such reactions, and may be selected from tetrahydrofuran, toluene, 1,4-dioxane, dichloromethane, or a combination thereof, preferably tetrahydrofuran. A molar ratio of the reducing agent to the compound of Formula II ranges from 1-5:1, and is preferably 2:1. The temperature for the reduction reaction ranges from -70°C to 80°C, preferably from 30°C to 70°C.

In step (3), the amino group protection is a reaction common in the art. This reaction is carried out in the presence of a base, for example, triethylamine, at 0°C to 40°C. The reagent for providing the amino protecting group is preferably selected from di-tert-butyl dicarbonate or 9-fluorenylmethyl chloroformate, more preferably di-tert-butyl dicarbonate. The reaction solvent is a solvent commonly used for such reactions, and may be selected from 1,4-dioxane, dichloromethane, dimethyl sulfoxide, toluene, or tetrahydrofuran.

In step (4), in some specific examples, the reaction for introducing the aldehyde group into the compound of Formula IV is carried out in the presence of a strong base. The strong base reagent is preferably selected from n-butyllithium, tert-butyllithium, or lithium metal, more preferably n-butyllithium. The reaction temperature ranges from -80°C to -60°C, and a molar ratio of the strong base reagent to the compound of Formula IV ranges from 1-5:1. The solvent used for the reaction for introducing the aldehyde group may be selected from tetrahydrofuran, methyltetrahydrofuran, or toluene.

In some other preferred examples, the reaction for introducing the aldehyde group into the compound of Formula IV is carried out in the presence of a metal catalyst, the metal catalyst is a palladium-containing catalyst, and the palladium-containing catalyst is preferably selected from palladium acetate, tetrakis(triphenylphosphine)palladium, Pd(dppf)Cl₂, or Pd(dba)₂. The reaction temperature ranges from -80°C to -60°C. An amount of the palladium catalyst in this reaction ranges from 0.002 to 0.1 eq. The solvent used for the reaction may be selected from toluene or tetrahydrofuran.

### Method for Synthesizing Elacestrant

A method for synthesizing Elacestrant includes the following steps:
(1) reacting a compound of Formula V with a compound of Formula VI, and reducing a generated product by a reducing agent to generate a compound of Formula VII, where the reducing agent further provides an ethyl group, and a reaction equation is as follows: 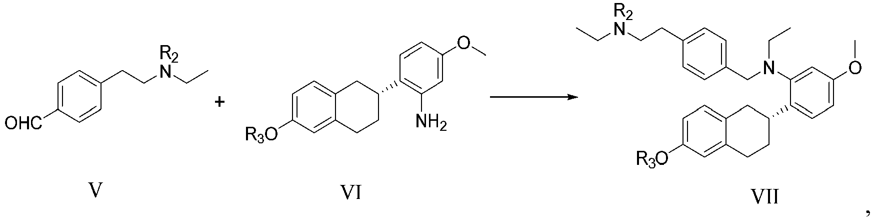 where in the reaction equation, R₂ is a nitrogen protecting group, and R₃ is H or a hydroxy protecting group; and
(2) removing a protecting group from the compound of Formula VII to afford Elacestrant, where the protecting group is a nitrogen protecting group and a hydroxy protecting group, or is a nitrogen protecting group,
where in step (1), the compound of Formula V reacts with the compound of Formula VI to generate a Schiff base, the Schiff base is reduced by the reducing agent, and an ethyl group is introduced to afford the compound of Formula VII, where the reducing agent is preferably selected from sodium triacetoxyborohydride. The reaction for generating the Schiff base is carried out at 50°C to 80°C, preferably from 60°C to 70°C. The solvent used in this step may be selected from tetrahydrofuran, toluene, 1,4-dioxane, methyltetrahydrofuran, or a combination thereof, preferably tetrahydrofuran. A molar ratio of the compound of Formula VI to the compound of Formula V ranges from 1:1-1.2.

The reaction for reducing the Schiff base and introducing the ethyl group is carried out at 40°C to 70°C, preferably from 50°C to 60°C. Preferably, after the compound of Formula V reacts with the compound of Formula VI to generate the Schiff base, the reaction solution is concentrated until distillation substantially ceases, and then the solvent and the reducing agent are added to reduce the Schiff base and introduce the ethyl group.

In step (2), when R₃ is a hydroxy protecting group, the hydroxy protecting group and the nitrogen protecting group are removed from the compound of Formula VII to afford Elacestrant; and when R₃ is H, the nitrogen protecting group is removed from the compound of Formula VII to afford Elacestrant. The removal of the hydroxy protecting group and the nitrogen protecting group may be carried out according to conventional methods for the respective protecting groups. For example, the nitrogen protecting group Boc may be removed under acidic conditions, for example, by adding triethylamine; and the hydroxy protecting group Bn may be removed in the presence of Pd/C. There is no temporal restriction on the removal of the hydroxy protecting group and the nitrogen protecting group. The hydroxy protecting group may be removed first followed by the nitrogen protecting group, or conversely, the nitrogen protecting group may be removed first followed by the hydroxy protecting group.

In a specific example of the present invention, a method for synthesizing Elacestrant includes the following steps:
(1) subjecting a compound of Formula I to a condensation reaction with ethylamine to afford a compound of Formula II;
(2) reducing the compound of Formula II to afford a compound of Formula III;
(3) protecting an amino group in the compound of Formula III to afford a compound of Formula IV; and
(4) converting R₁ in the compound of Formula IV into an aldehyde group to afford a compound of Formula V;
(5) reacting a compound of Formula V with a compound of Formula VI, and reducing a generated product by a reducing agent to generate a compound of Formula VII, where the reducing agent further provides an ethyl group; and
(6) removing a protecting group from the compound of Formula VII to afford Elacestrant, where the protecting group is a nitrogen protecting group and a hydroxy protecting group, or is a nitrogen protecting group,

where a reaction equation is as follows: 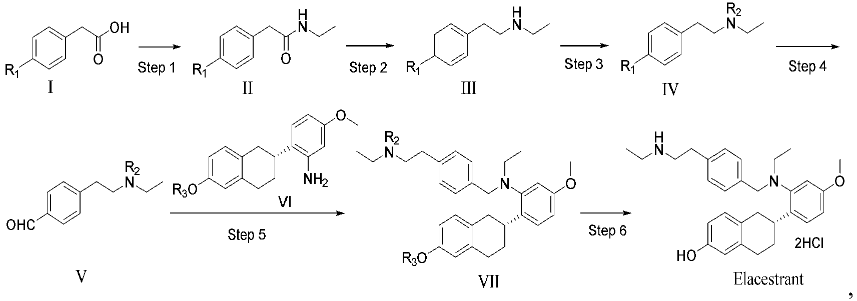
where in the reaction equation, R₁ is bromine or iodine, R₂ is a nitrogen protecting group, and R₃ is H or a hydroxy protecting group.

Compared with the prior art, the advantageous effects of the method for synthesizing Elacestrant according to the present invention are as follows:
1) it features short reaction steps, is concise and efficient, and is easy for industrialization;
2) early introduction of the amino group avoids the risks of low yield and product racemization caused by amide reduction in the final step;
3) all reaction steps involved are conventional reactions with low-cost starting materials, significantly reducing the API cost;
4) each reaction step involved achieves high yield; for example, the six-step synthesis starting from 4-bromophenylacetic acid affords Elacestrant in an overall yield as high as 61.7%, which is significantly higher than those reported in the prior art, and the final product purity is greater than 99%.

The present invention will be further described in detail below with reference to specific examples. The scope of protection of the present invention is not limited to the following examples. Unless specifically mentioned, the procedures, conditions, reagents, experimental methods, and the like described in the following examples are common general knowledge and well-known techniques in the art, and the present invention imposes no particular limitations in this regard. Unless otherwise specified, the raw materials used in the present invention are commercially available or may be prepared according to methods reported in the prior art. For example, the compound of Formula VI-1 mentioned below may be prepared according to the method reported in the prior art WO2020167855.

### Example 1: Synthesis of Compound V-1 (in compound of Formula V, R₂ = Boc)

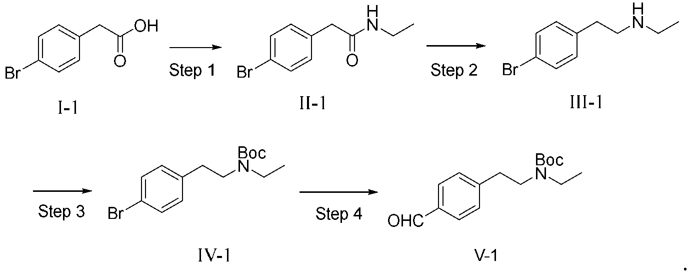

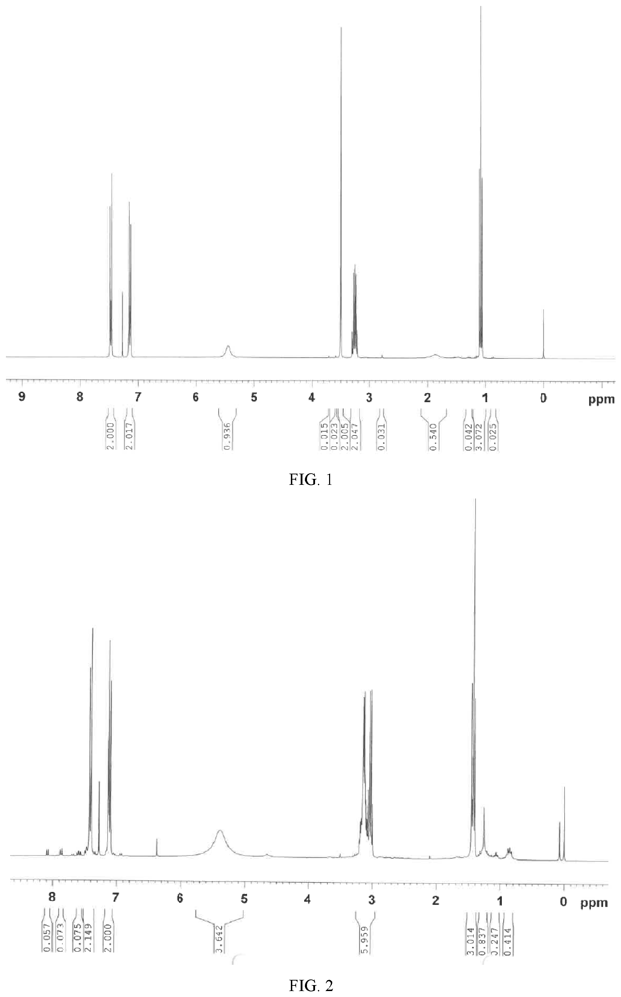

In step 1, DMF (0.4 L), dichloromethane (0.8 L), and a compound of Formula I-1 (200.0 g) were added into a reactor; the temperature was decreased to 0°C to 10°C; ethylamine hydrochloride (91.0 g) and EDCI (214.0 g) were added; the temperature was controlled at 0°C to 10°C. DIPEA (282.5 g) was then added dropwise; the temperature was increased to 20°C to 30°C; after the raw materials were completely converted, concentration was performed; water was added to the concentrate to precipitate a product; cooling, filtration, and drying were performed to afford 212.1 g of a compound of Formula II-1, with yield of 94.5% (FIG. 1: ¹H-NMR).

In step 2, tetrahydrofuran (0.9 L) and the compound of Formula II-1 (180.0 g) were added into the reactor; the system temperature was controlled at T ≤ 30°C; a 2.0 M borane-dimethyl sulfide complex solution (929 mL) was added dropwise; then the temperature was increased to 50°C to 60°C, after the raw materials were completely converted, the system was cooled; water was added to the mixture, followed by extraction with ethyl acetate; an organic phase was washed once with saturated aqueous sodium chloride solution and then concentrated to dryness to afford 60.0 g of an oily product compound of Formula III-1, with yield: of 94.8% (FIG. 2: ¹H-NMR).

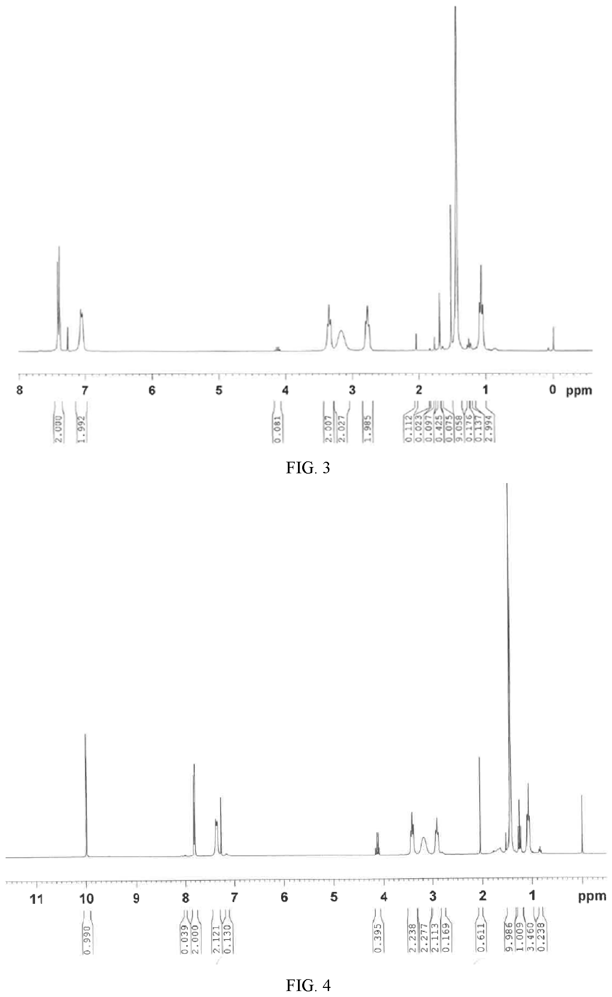

In step 3, 1,4-dioxane (0.8 L), the compound of Formula III-1 (160.0 g), triethylamine (108.4 g), and di-tert-butyl dicarbonate (186.9 g) were added into the reactor; the reaction was monitored by TLC until the raw materials were completely converted; concentration was performed; water was added to the concentrate, followed by the addition of ethyl acetate and extraction twice; an organic phase was washed once with a saturated aqueous sodium chloride solution and concentrated to dryness to afford 217.1 g of an oily product compound of Formula IV-1, with yield of 93.3% (FIG. 3: ¹H-NMR).

In step 4, tetrahydrofuran (1.1 L) and the compound of Formula IV-1 (217.0 g) were added into the reactor; the temperature was decreased to -78°C; n-butyllithium (296 mL) was added dropwise; the temperature was controlled at -80°C to 75°C; stirring was performed for 0.5 h to 1 h; N,N-dimethylformamide (162.3 g) was added dropwise; the temperature was controlled at -80°C to 75°C for reaction; after the raw materials were completely converted, a saturated aqueous ammonium chloride solution was added dropwise to the system, followed by the addition of water, stirring, and liquid separation; an aqueous phase was extracted with ethyl acetate; organic phases were combined, washed once with a saturated aqueous sodium chloride solution, and concentrated to dryness to afford 161.9 g of an oily product compound of Formula V-1, with yield of 87.2% (FIG. 4: ¹H-NMR).

### Example 2: Synthesis of Elacestrant

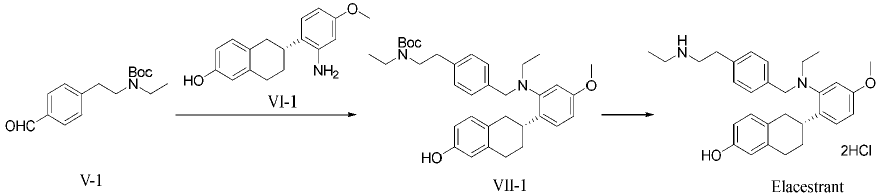

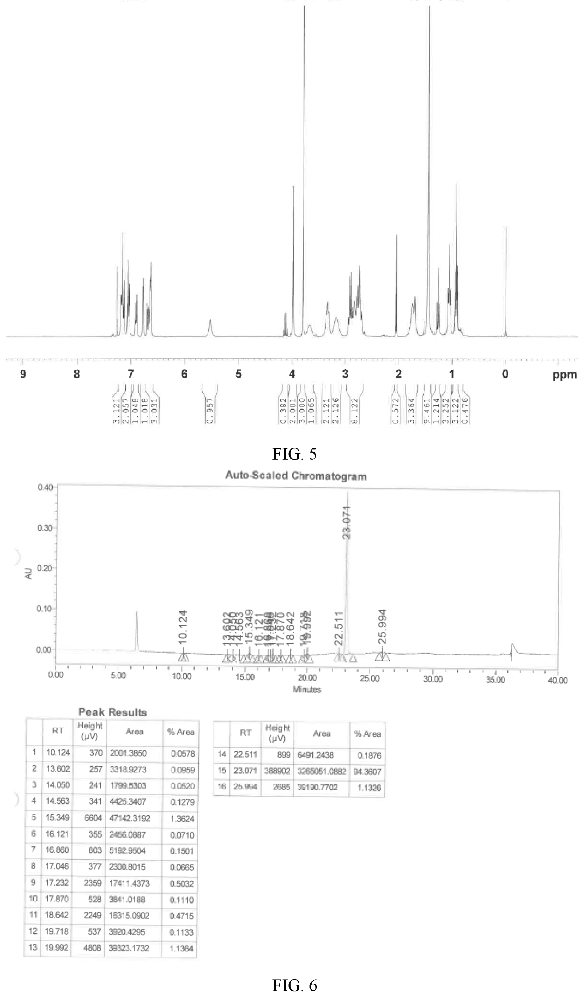

Tetrahydrofuran (1.5 L), n-heptane (0.75 L), a compound of Formula VI-1 (in a compound of Formula VI, R₃ = H) (100.0 g), a compound of Formula V-1 (123.4 g), and D-dibenzoyl tartaric acid (0.1 g) were added into a reactor; the temperature was increased to 60°C to 70°C for reaction; the reaction was monitored until the raw materials were completely converted, concentration was performed until distillation substantially ceased; tetrahydrofuran (1.5 L) and sodium triacetoxyborohydride (354.1 g) were then added; the temperature was increased to 50°C to 60°C for reaction; the reaction was monitored until the raw materials were completely converted, the system was cooled to 20°C to 30°C, followed by the addition of water and liquid separation; an aqueous phase was extracted with ethyl acetate (5 times the volume of water); and organic phases were combined and concentrated to dryness to afford a crude product. The crude product was purified by silica gel column chromatography to afford 84.5 g of an oily product compound of Formula VII-11, with yield: 89.2% and HPLC purity of 94.36% (FIG. 5: ¹H-NMR, FIG. 6: HPLC).

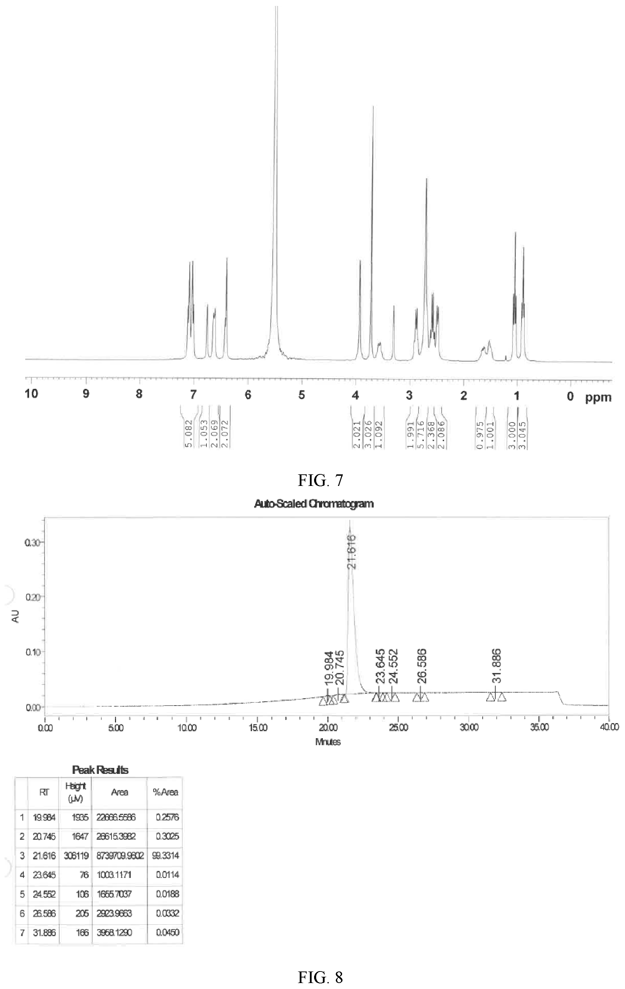

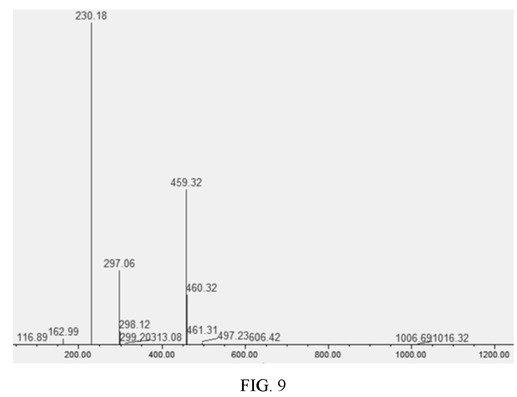

Ethyl acetate (1.0 L), the compound of Formula VII-1 (100.0 g, 179.2 mmol), and a 2 M ethyl acetate solution of hydrochloric acid (448 mL) were added into the reactor; the temperature was controlled at 20°C to 30°C for reaction; after the raw materials were completely converted, the temperature was decreased to 0°C to 10°C, followed by filtration and drying to afford 90.4 g of a solid, with yield of 95.0% and HPLC purity of 99.33% (FIG. 7: ¹H-NMR, MeOH+NaOD, FIG. 8: HPLC, FIG. 9: mass spectrum).

### Example 3: Synthesis of Elacestrant

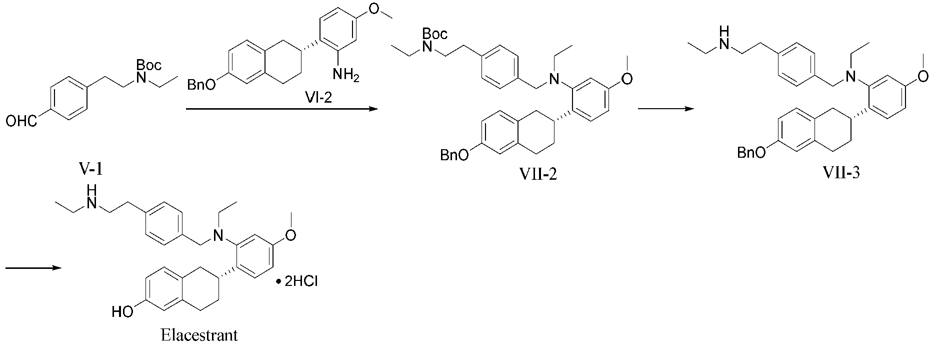

Methyltetrahydrofuran (1.4 L), n-heptane (1 L), a compound of Formula VI-2 (in a compound of Formula VI, R₃ = Bn) (135.3 g), a compound of Formula V-1 (125.1 g), and D-dibenzoyl tartaric acid (0.15 g) were added into a reactor; the temperature was increased to 70°C to 80°C for reaction; after the raw materials were completely converted, concentration was performed; methyltetrahydrofuran (2 L) and sodium triacetoxyborohydride (377.1 g) were then added; the temperature was increased to 60°C to 70°C for reaction; after the raw materials were completely converted, the temperature was decreased to 20°C to 30°C, followed by the addition of water into the reaction solution and liquid separation; an aqueous phase was extracted with isopropyl acetate; and organic phases were combined and concentrated to dryness to afford a crude product. The crude product was purified by silica gel column chromatography to afford 200.5 g of an oily product compound of Formula VII-2, with yield of 83.2%.

The compound of Formula VII-2 (120 g, 1.0 eq.) and methanol (1.2 L) were added into the reactor; the temperature was decreased to 0°C to 10°C; trifluoroacetic acid (63.3 g, 3.0 eq.) was added dropwise; after the reaction was completed, concentration was performed, followed by the addition of water; the pH was adjusted to 9 to 10 by using an aqueous NaOH solution; filtration and vacuum drying were performed to afford a compound of Formula VII-3 (91.8 g, yield: 90.4%).

The compound of Formula VII-3 (91.7 g), methanol (0.92 L), and 5% Pd/C (2.3 g, 5 wt%) were added into a hydrogenation reactor; a reaction was carried out at 30 psi to 40 psi for 8 h to 10 h; after the reaction was completed, filtration was performed; an aqueous HCl solution (42.3 g) was added into the filtrate; concentration was performed to 5 to 6 volumes, followed by filtration and vacuum drying to afford 83.4 g of Elacestrant, with yield of 94.2%.

### Example 4: Synthesis of Elacestrant

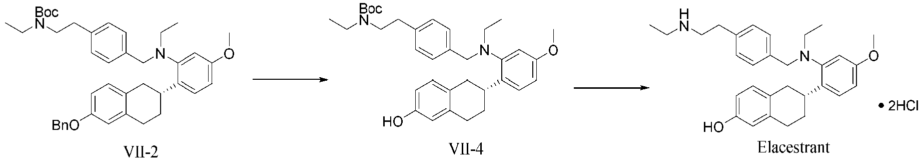

A compound of Formula VII-2 (120 g, 0.185 mol, 1.0 eq.), methanol (1.2 L, 10 volumes), and 10% Pd/C (1.5 g, 2.5 wt%) were added into a hydrogenation reactor, a reaction was carried out under 35 psi to 45 psi at 40°C to 45°C; after the reaction was completed, filtration was performed and the filtrate was concentrated to afford 123.1 g of a crude product compound of Formula VII-4.

The crude product compound of Formula VII-4 (123.1 g) was added into a reaction flask; an aqueous HCl solution (93.8 g) was added at room temperature; the mixture was heated to 50°C to 60°C for reaction for 3 h to 4 h; after it was monitored by TLC that the reaction was completed, concentration was performed to 5 volumes, followed by filtration and vacuum drying to afford 84.2 g of a product, with yield of 85.9%.

All documents cited in the present invention are incorporated herein by reference as if each document were individually incorporated by reference. In addition, it should be understood that after reading the above teachings of the present invention, those skilled in the art may make various alterations or modifications to the present invention. These equivalent forms also fall within the scope defined by the appended claims of the present application.

## Claims

1. A method for synthesizing Elacestrant, comprising the following steps:
(1) reacting a compound of formula V with a compound of formula VI, and reducing a generated product by a reducing agent to generate a compound of Formula VII, wherein the reducing agent further provides an ethyl group, and a reaction equation is as follows: 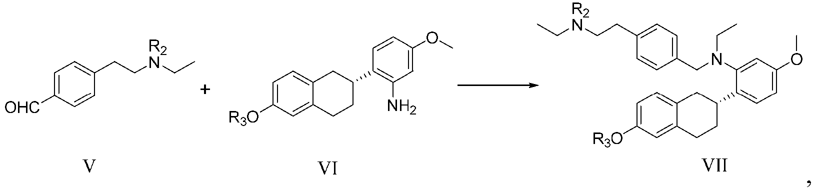 where in the reaction equation, R₂ is a nitrogen protecting group, and R₃ is H or a hydroxy protecting group; and
(2) removing a protecting group from the compound of Formula VII to afford Elacestrant, wherein the protecting group is a nitrogen protecting group and a hydroxy protecting group, or is a nitrogen protecting group.

2. The method for synthesizing Elacestrant according to claim 1, wherein in step (1), the compound of Formula V reacts with the compound of Formula VI at 50°C to 80°C to generate a Schiff base, then the Schiff base is reduced by the reducing agent at 40°C to 70°C, and an ethyl group is introduced to afford the compound of Formula VII.

3. The method for synthesizing Elacestrant according to claim 2, wherein
the reaction for generating the Schiff base is carried out in a solvent selected from:
toluene, tetrahydrofuran, methyltetrahydrofuran, or a combination thereof,
and/or
the reaction for reducing the Schiff base and introducing the ethyl group is carried out in a solvent selected from:
toluene, tetrahydrofuran, methyltetrahydrofuran, or a combination thereof,
and/or
a molar ratio of the compound of Formula VI to the compound of Formula V ranges from 1:1 to 1.2.

4. The method for synthesizing Elacestrant according to claim 1, wherein the reducing agent is selected from sodium triacetoxyborohydride.

5. The method for synthesizing Elacestrant according to claim 1, wherein the nitrogen protecting group is selected from Boc or Fmoc, and/or
the hydroxy protecting group is selected from Bn, Cbz, or Ac.

6. The method for synthesizing Elacestrant according to claim 1, wherein in step (2),
when R₃ is H, the nitrogen protecting group is removed from the compound of Formula VII to afford Elacestrant; and
when R₃ is a hydroxy protecting group, the nitrogen protecting group and the hydroxy protecting group are sequentially removed from the compound of Formula VII, or the hydroxy protecting group and the nitrogen protecting group are sequentially removed from the compound of Formula to afford Elacestrant.

7. The method for synthesizing Elacestrant according to claim 1, wherein a method for synthesizing the compound of Formula V comprises the following step: converting R₁ in the compound of Formula IV into an aldehyde group to afford the compound of Formula V, wherein a reaction equation is as follows: 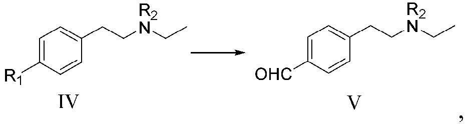 wherein in the reaction equation, R₁ is selected from bromine or iodine, and R₂ is a nitrogen protecting group.

8. The method for synthesizing Elacestrant according to claim 1, wherein converting R₁ in the compound of Formula IV into an aldehyde group is carried out in the presence of a strong base reagent or a metal catalyst,
the strong base reagent is selected from n-butyllithium, tert-butyllithium, or lithium metal, preferably n-butyllithium,
the metal catalyst is a palladium-containing catalyst, and the palladium-containing catalyst is selected from palladium acetate, tetrakis(triphenylphosphine)palladium, Pd(dppf)Cl₂, or Pd(dba)₂.

9. A method for synthesizing a compound of Formula V, comprising the following steps:
(1) subjecting a compound of Formula I to a condensation reaction with ethylamine to afford a compound of Formula II;
(2) reducing the compound of Formula II to afford a compound of Formula III;
(3) protecting an amino group in the compound of Formula III to afford a compound of Formula IV; and
(4) converting R₁ in the compound of Formula IV into an aldehyde group to afford a compound of Formula V,
wherein a reaction equation is as follows: 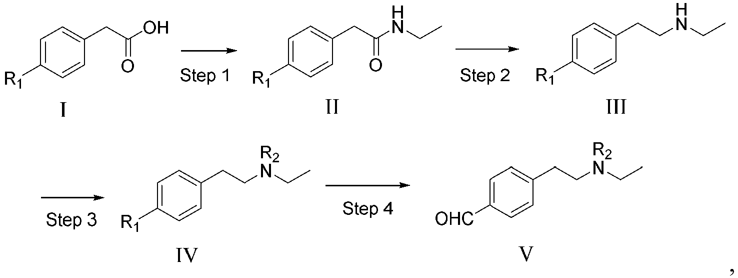
wherein in the reaction equation, R₁ is selected from bromine or iodine, and R₂ is a nitrogen protecting group.

10. The method for synthesizing the compound of Formula V according to claim 9, wherein in step (1), the condensation reaction is carried out in the presence of a condensing agent, and the condensing agent is selected from EDCI, DCC, HOBt, CDI, HBTu, or a combination thereof.

11. The method for synthesizing the compound of Formula V according to claim 9, wherein in step (2), the reducing agent used in the reduction reaction is selected from borane, a borane complex, lithium aluminum hydride, sodium borohydride, sodium borohydride/Lewis acid, or a combination thereof, preferably borane.

12. A method for synthesizing Elacestrant, comprising the following steps:
(1) reacting a compound of Formula V with a compound of Formula VI, and reducing a generated product by a reducing agent to generate a compound of Formula VII, wherein the reducing agent further provides an ethyl group, and a reaction equation is as follows: 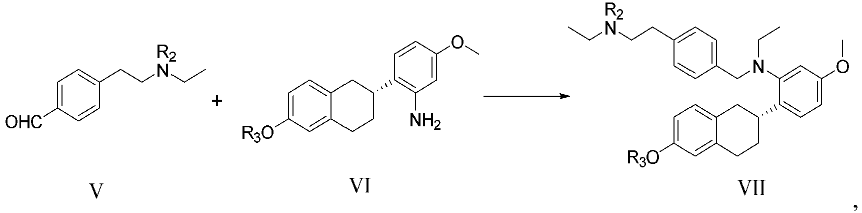 wherein in the reaction equation, R₂ is a nitrogen protecting group, and R₃ is a hydroxy protecting group; and
(2) removing a protecting group from the compound of Formula VII to afford Elacestrant, wherein the protecting group is a nitrogen protecting group and a hydroxy protecting group, or is a nitrogen protecting group,
wherein the compound of Formula V is obtained by adopting the method according to any one of claims 9 to 11.
